# NEUE EUROPÄISCHE PATENTSCHRIFT

(11) **EP 0 439 846 B2**
(45) Veröffentlichungstag und Bekanntmachung des Hinweises auf die Entscheidung über den Einspruch: **28.10.1998**
(45) Hinweis auf die Patenterteilung: 02.02.1994
(21) Anmeldenummer: 90125845.9
(22) Anmeldetag: 31.12.1990
(51) Int. Cl.: C08G 73/14, A61K 9/52

(54) **Biologisch abbaubare Polymere, Verfahren zu ihrer Herstellung und Verwendung derselben für Depotzubereitungen mit kontrollierter Wirkstoffabgabe**
Biodegradable polymers, a process for their preparation, and the use thereof for depot formulations with controlled delivery of active ingredient
Polymères biodégradables, procédé de leur préparation et leur utilisation comme support de médicament à libération contrôlée

(30) Priorität: 31.01.1990 DE 4002736
(43) Veröffentlichungstag der Anmeldung: 07.08.1991
(73) Patentinhaber: HOECHST AKTIENGESELLSCHAFT, 65926 Frankfurt am Main (DE)
(72) Erfinder: Krone, Volker, Dr., W-6093 Flörsheim (DE); Walch, Axel, Dr., W-6000 Frankfurt am Main (DE)

(56) Entgegenhaltungen:
- EP-A- 256 366
- EP-A- 406 623
- EP-A- 0 274 127
- DE-A- 2 032 470
- US-A- 3 864 380
- J. Org. Chem., vol. 24, 1959, pp 1662-1666
- Biosystems, vol. 7, 1975, pp 213-221
- Biopolymers, vol. 20, 1989, pp. 1605-1614

## Beschreibung

Die Erfindung betrifft biologisch abbaubare Polymere, insbesondere Polyaminodicarbonsäure-co-anhydroaminodicarbonsäure-derivate, ein Verfahren zu ihrer Herstellung und die Verwendung derselben für Depotzubereitungen mit kontrollierter Wirkstoffabgabe. Dabei werden die Wirkstoffe in einer Matrix, bestehend aus den erfindungsgemäßen Polymeren, eingebettet und in vivo durch Bioerosion der Matrix kontrolliert freigesetzt. Beim Abbau der erfindungsgemäßen Produkte entstehen überwiegend körpereigene, bzw. in ihrer Bioverträglichkeit bekannte Fragmente, die in natürlichen Stoffwechselwegen metabolisiert oder auf Grund ihrer Wasserlöslichkeit durch die Nieren ausgeschieden werden.

Eine moderne Arzneitherapie erfordert insbesondere zurApplikation von Wirkstoffen neue Darreichungsformen, die eine kontrollierte Abgaberate der Wirkstoffe mit hoher Biokompatibilität des Depots vereinigen. Eine langandauernde kontrollierte Wirkstoffabgabe ist wegen der zunehmenden Bedeutung chronischer Erkrankungen und langzeitorientierter Therapiekonzepte in Human- und Veterinärmedizin von großer Aktualität. Als Matrixmaterialien für solche Depotsysteme sind bioabbaubare Polymere besonders vorteilhaft, da die Bioerosion die Wirkstofffreisetzung steuert und die chirurgische Entfernung eines derartigen Depots entbehrlich macht.

Arzneiabgabesysteme, in denen der Wirkstoff in einer nicht abbaubaren Polymermatrix dispergiert ist und durch Diffusion freigesetzt wird, sind in der amerikanischen Patentschrift 4,069,307 beschrieben. Nach Erschöpfung des Wirkstoffreservoirs müssen solche Implantate jedoch operativ aus dem Organismus entfernt werden.

In biologisch abbaubaren Arzneistoffabgabesystemen, wie in dem amerikanischen Patent 4,093,709 angegeben, wird der Wirkstoff in einem bioabbaubaren Polymeren dispergiert, welches beim Abbau den Wirkstoff freigibt. Typische, nach dem Stand der Technik meist untersuchte, biologisch abbaubare Polymere sind Homo- und Copolyester, insbesondere der Milch- und Glykolsäure, wie sie in den US-Patenten 3,773,919 bzw. 3,297,033 beschrieben sind. Nachteilig ist u.a. die geringe oder schlecht kontrollierbare Quellbarkeit der Polyester im physiologischen Milieu, welche den Transport der im Implantat inkorporierten Wirkstoffe durch die Polymermatrix hindurch an die Oberfläche behindert und eine nach initialem "burst effect" nur geringe Freisetzungsrate bewirkt.

In neuerer Zeit sind Polyacetale und -ketale (US-PS 4,304,767) bzw. Polyanhydride (H.G. Rosen et al., Biomaterials 4, 131 (1983)) und Polyorthoester (US-PS 4,180,646) beschrieben worden, die als biologisch abbaubare Polymere für den Einsatz als Implantatmaterialien entwickelt wurden.

Als weitere Polymerklasse sind in der amerikanischen Patentschrift 3,371,069 Polyamide, insbesondere Poly-a-L-Aminosäuren, als bioresorbierbare Implantatmaterialien beschrieben worden. Die technische Herstellung von Polyaminosäuren erfordert jedoch den Einsatz teurer geschützter Aminosäuren, größere Mengen an hochgiftigem Phosgen, die Abspaltung der Schutzgruppen und die anschließende Derivatisierung der erhaltenen Polymere.

Ein weiterer Nachteil derartiger Polyamide ist das Vorliegen geladener Gruppen durch nicht vollständige Derivatisierung beim Aufbau des Implantatmaterials. Die in den Patentschriften erwähnte reine Poly-L-Glutaminsäure sowie reines Poly-L-Lysin sind toxikologisch äußerst bedenklich (A.D. Kenny, Proc. Soc. Exp. Biol. Med. 100, 778 (1959)) und ihre durch Bioabbau aus hydrophoben Derivaten entstehenden Copolymere müssen daher ebenfalls sehr kritisch bewertet werden.

In der US-PS 4 356 166 werden bioabbaubare Implantatmaterialien beschrieben, die in vivo eine bioaktive Verbindung freisetzen. Als bioaktive Verbindungen werden in der US-PS 4 356 166 Progestine beschrieben, die zunächst chlorformyliert und dann kovalent an das Polymer gebunden werden. Als Polymere werden dabei Poly-(hydroxyalkyl)-L-glutamin oder Poly(hydroxyalkyl)-L-aspartamid eingesetzt. Die bioaktiven Verbindungen sind entweder über eine sogenannte "Spacer-Group" oder aber direkt über die reaktive Komponente des Polymeren gebunden. Die Freigaberate der bioaktiven Verbindung wird über das Molekulargewicht des Polymeren oder über die Länge und den Charakter der "Spacer-Group" gesteuert.

Nachteilig an den Substanzen gemäß US-PS 4 356 166 ist, daß sie bereits selbst Pharmaka mit hoher pharmakologischer Aktivität darstellen. In solchen Polymer/Wirkstoff-Konjugaten (polymeric drugs) bilden biokompatibles Polymer und Wirkstoff eine Einheit, deren Eigenschaften in komplexer Weise von beiden Komponenten bestimmt werden. Die Freigaberate des polymerfixierten bioaktiven Moleküls ist im Rahmen dervorgenannten Parameter variabel, jedoch entscheidend von der Natur des Wirkstoffs abhängig. Hydrophobe bioaktive Stoffe, wie z.B. Steroidhormone, können im wäßrigen biologischen Milieu nur sehr langsam vom Polymerrückgrat abgespalten werden und eignen sich daher ausschließlich für extreme Langzeitdepotformen. Für jeden Wirkstoff müssen neue Polymer/Wirkstoff-Konjugate synthetisiert werden, was die Verwendbarkeit des in der US-PS 4 356 166 beschriebenen Konzeptes polymerfixierter Pharmaka außerordentlich eingeschränkt.

Aus diesen Gründen eignen sich diese Substanzen nicht für den Einsatz als gesteuert abbaubare Polymere, die durch ihre eigene Biodegradation einen Wirkstoff freisetzen, der in die inerte Polymermatrix eingebettet ist, ohne dabei chemisch an das Polymere gebunden zu sein.

Eine wesentliche Verbesserung wurde bereits durch die bioabbaubaren Poly-(hydroxyalkyl)-aminodicarbonsäu- rederivate, die in der EP 0 274 127 beschrieben sind, erreicht. Jedoch weisen auch diese bioabbaubaren Polymere noch folgende Nachteile auf:
a) Die mit diesen Substanzen hergestellten Mikropartikel sind nur mit Hilfsmitteln in Wasser suspendierbar. Die Verwendung von Hilfsmitteln wie ®Pluronic oder Dextranen ist jedoch nicht unproblematisch, da diese Hilfsmittel bei der Implantation der Wirkstoffdepots in den Organismus gelangen.
b) Im wäßrigen Medium haben solche Mikropartikel eine klebrige, schmierige Konsistenz, die zu einer Zusammenlagerung der einzelnen Teilchen führt.
c) Das Freigabeprofil des Wirkstoffs aus diesen Mikropartikeln - unter Verwendung einiger Poly(hydroxyalkyl)-aminodicarbonsäuren gemäß EP 0 274127 zeigt immer noch einen erheblichen "initial burst", d. h. eine anfänglich sehr hohe Freisetzungsrate des Wirkstoffs, die dann sehr schnell abnimmt.
d) Die für Mikropartikel gemäß EP 0 274 127 in vitro ermittelten Abbauzeiten (Phosphatpuffer, 37°C) sind - insbesondere für die Anwendung als Wirkstoffdepot für Peptidwirkstoffe - zu lang.

Aufgabe der vorliegenden Erfindung war es, Polymere zu entwickeln, die die in der EP 0 274 127 beschriebenen Vorteile aufweisen, ohne jedoch die oben aufgeführten Nachteile in Kauf nehmen zu müssen.

Es wurden nun Polyaminodicarbonsäure-co-anhydroaminodicarbonsäure-Derivate (Polyaminodicarbonsäure-co-AHADS-Derivate) synthetisiert, die sich überraschenderweise hervorragend zum Einsatz als abbaubare Arzneistoffimplantate mit kontrollierter Wirkstoffabgabe eignen. Die Wirkstoffe werden dabei nicht chemisch an das Polymere gebunden, sondern lediglich in diese Polymermatrix eingebettet. Durch Einbau geeigneter biologisch inaktiver Gruppen läßt sich die Abbaugeschwindigkeit des Polymers in vivo in gewünschter Weise steuern und somit gleichzeitig die Freigaberate des Wirkstoffs. Durch den Einbau ungeöffneter Anhydroaminodicarbonsäurednge (AHADS-Ringe) verbessert sich insbesondere die Suspendierbarkeit der mit den erfindungsgemäßen Polymeren hergestellten Mikropartikel in Wasser sowie deren Konsistenz. Außerdem wird das Freigabeprofil sowie die Abbauzeit weiter verbessert. Der Vorteil dieser Verfahrensweise ist, daß nun auch solche Wirkstoffe über einen längeren Zeitraum mit relativ konstanter Dosis appliziert werden können, die entweder überhaupt nicht chemisch an ein Polymer gebunden werden können, oder aber zu empfindlich sind, um die doch recht drastischen Bedingungen bei der chemischen Ankoppelung an das Polymer zu überstehen Darüber hinaus sind die Polymere als pharmakologisch inerte Matrix grundsätzlich für alle relevanten Pharmaka universell einsetzbar, unabhängig von der Molekülgröße und anderer physikalisch-chemischer Parameter. Daß durch den Einbau ungeöffneter AHADS-Ringe die Eigenschaften des Polymeren gemäß EP 0 274 127 weiter verbessert werden konnte, ist deshalb besonders überraschend, weil die Klebrigkeit der Polymere und die schlechte Suspendierbarkeit der Mikropartikel gemäß EP 0 274 127 offenbar auf der Hydrophilie und der Ausprägung von Wasserstoffbrücken der Polyamidhauptketten bzw. der amidhaltigen Seitengruppen beruht. Die in den erfindungsgemäßen Copolymeren verbliebenen AHADS-Einheiten sind aber mindestens ebenso zur Ausprägung von Wasserstoffbrücken befähigt wie die Polyamidhauptkette bzw. die amidhaltige Seitengruppe. Jedoch überwiegt unerwarteterweise der "steife" Charakter der AHADS-Einheiten, was zu festen, auch in Wasser nicht schmierenden Produkten führte. Die Teilchen lagern sich in Wasser auch kaum zusammen, so daß auf Suspensionshilfsmittel ganz verzichtet werden kann. Die hervorragende Konsistenz der erfindungsgemäßen Copolymere zeigt sich ebenfalls beim problemloseren Versprühen zu Mikropartikeln.

Einen weiteren wesentlichen Vorteil zeigen wirkstoffhaltige Mikropartikel aus diesen Copolymeren bei Releasemessungen: es tritt fast kein "initial burst", also keine stark erhöhte Wirkstofffreisetzung in Kontakt mit Wasser in den ersten Stunden auf.

Auch die schnelleren "Abbauzeiten", die auf eine raschere Umsetzung bzw. Auflösung in Wasserzurückzuführen ist « 4 Wochen im Phosphatpuffer bei 37°C), kommt der Anwendung als Wirkstoffdepot für Peptidwirkstoffe, die innerhalb 4 Wochen weitgehend freigesetzt werden sollen, entgegen.

Die erfindungsgemäßen copolymeren Produkte sind außerdem kostengünstiger, da man nur partiell die erste Stufe der Synthese weiter umsetzen muß. Diese biologisch abbaubaren Polymere erhält man durch Polykondensation von Aminodicarbonsäuren, die anschließend teilweise mit Wasser, Aminoalkoholen, Ammoniak oder Alkoholen zu Poly- aminodicarbonsäure-co-AHADS-Derivaten umgesetzt werden und dann gegebenenfalls mit Carbonsäuren, Carbonsäurehalogeniden oder Halogenameisensäureestern in einer polymeranalogen Acylierung zu weiteren erfindungsgemäßen Polyaminodicarbonsäure-co-AHADS-Derivaten reagieren. In vivo werden diese Polymere zu untoxischen, nicht allergenen und nicht immunogenen Verbindungen metabolisiert und ausgeschieden.

Die Erfindung betrifft somit:
Polyaminodicarbonsäure-co-anhydroaminodicarbonsäure-Derivate der Formel 1 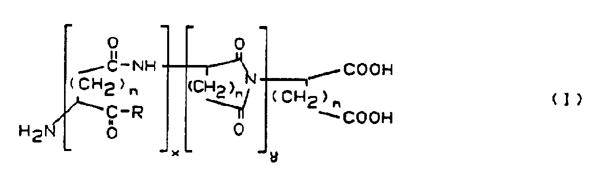 in der
   n 1 oder 2
   x 1 bis 500
   y 1 bis 500 ist, wobei
   x + y 2 bis 1000 ist und
   R O-R¹ oder NH-R² bedeutet, worin
   R² H, (CH₂)m-OR₁, (CH₂)ₘ-O-C(O)-R¹ oder (CH₂)ₘ-O-C(O)-OR¹ bedeutet und
   m 2 bis 6 ist und
   R^{l} H, Aryl, Aralkyl, Arylalkenyl, Alkyl oder C₃-C₈-Cycloalkyl oder ein biologisch inaktiver Steroidalkoholoder ein Aminosäur-Rest bedeutet, wobei Aryl unsubstituiert ist oder substituiert ist mit C₁-C₄-Alkyl, C₂-C₄-Alkenyl, C₁-C₄-Alkylcarbonyloxy, C₁-C₄-Alkoxycarbonyl, C₁-C₄-Alkoxy oder Hydroxy

   wobei die für R¹ genannten Alkylreste 1 - 22 C-Atome und die Alkenylreste 2 - 22 C-Atome aufweisen, die nicht unterbrochen oder durch eine Carbonyloxy-oder Oxycarbonylgruppe unterbrochen sind, wobei die in eckige Klammern gesetzten Wiederholungseinheiten statistisch und/oder in Blöcken im Polymeren verteilt sind und wobei sowohl die mit x als auch die mit y gekennzeichneten Wiederholungseinheiten identisch oder unterschiedlich sind und wobei die Aminosäuren a- und/oder ß-verknüpft sind, wobei eine Verbindung der Formel 1, bei der n 1, m 2, R-NHR², R²⁻(CH₂)ₘ-OR¹, R¹-H und x gleich y ist, ausgeschlossen ist und wobei eine Verbindung der Formel I, bei der n 1 und R-OH bedeuten und x und y die in Formel 1 genannte Bedeutung haben, ausgeschlossen ist.

Unter Aryl werden aromatische Kohlenwasserstoff-Reste verstanden wie Phenyl und Naphthyl, insbesondere Phenyl. Bei den angegebenen substituierten Arylresten sind 1 bis alle ersetzbaren Wasserstoffatome durch identische oder unterschiedliche Substituenten ersetzt. Bevorzugt sind die Arylreste mono- oder disubstituiert. Die genannten Alkyl- und Alkenylreste können sowohl geradkettig als auch verzweigt sein Die biologisch inaktiven Steroidalkohole sind bevorzugt über ihre OH-Gruppe gebunden. Ein bevorzugter Stemidalkohol ist Cholesterol. Bei den für R¹ genannten Aminosäure-Resten handelt es sich bevorzugt um natürlich vorkommende Aminosäuren wie Tyr, Ala, Ser oder Cys, besonders bevorzugt um Tyr und Ala. Sie können sowohl über ihre NH₂-als auch über ihre COOH-Funktion gebunden sein.

Ebenso betrifft die Erfindung das Verfahren zur Herstellung der obengenannten Polyamide und deren Verwendung auch in Abmischung mit anderen, bioverträglichen Polyamiden, besonders in Verbindung mit biologisch aktiven Substanzen, als abbaubare Wirkstoffdepotzubereitung mit kontrollierter Wirkstoffabgabe.

Im folgenden wird die Erfindung detailliert beschrieben.

Als Aminodicarbonsäuren werden Asparaginsäure und/oder Glutaminsäure eingesetzt, die in einer Polykondensationsreaktion zu den entsprechenden Polyanhydroaminodicarbonsäuren II reagieren. Durch partielle Umsetzung mit einer oder mehreren Verbindungen der Formeln III oder IV oder NH₃ 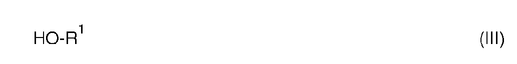 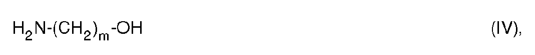 worin m und R¹ wie oben zu Formel definiert sind, erhält man einen α,β-Poly-D,L-Aminosäureester oder ein a,ß-Poly-D,L-Aminosäureamid der Formel VIII 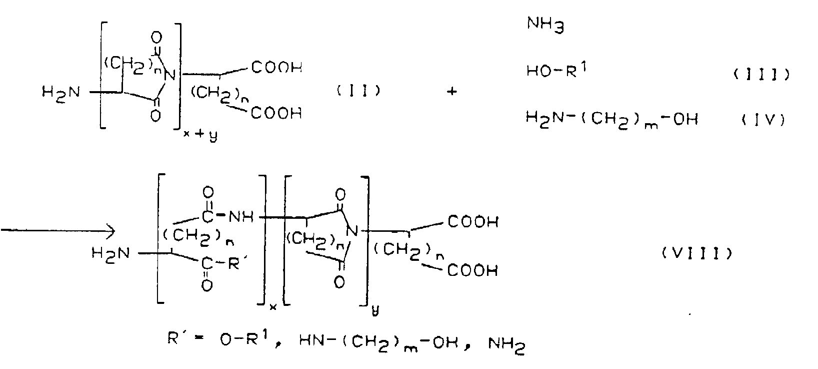 Erfindungswesentlich ist bei dieser Umsetzung, daß die Polyanhydroaminodicarbonsäure (II) nur teilweise in die offenkettigen Derivate überführt wird. Der Anteil an ungeöffneten Anhydroaminodicarbonsäure-Einheiten beträgt bevorzugt 10 bis 90 %, besonders bevorzugt 60 bis 80 % (die Prozentangaben beziehen sich auf die Gesamtzahl der Wiederholungseinheiten im Gesamtpolymeren). In Abhängigkeit davon, nach welcher Seite der Anhydroaminodicarbonsäurering bei der oben beschriebenen Reaktion geöffnet wird, erhält man a-bzw. β-verknüpfte Aminosäuren. Bevorzugt eingesetzte Verbindungen der Formeln III und IV sind: 2-Aminoethanol, 3-Aminopropanol, 2-Aminopropanol, Alkohole mit 1-18 C-Atomen, insbesondere Methanol, Ethanol, Isoamylalkohol, Tertiärbutylalkohol und Isopropylalkohol.

Ein Verfahren zur Herstellung von a,ß-Poly-(2-hydroxyethyl)-DL-Aspartamid (PHEA) (Formel I; y = O; R = NH-CH₂-CH₂-0H) wird von P. Neri, G. Antoni, F. Benvenuti, F. Cocola, G. Gazzei, in J. Med. Chem. Bd. 16; 893 (1973) beschrieben. Eine allgemeine Arbeitsvorschrift zur Herstellung von PHEAfindet sich in P. Neri, G Antoni, Macromol. Synth. Bd. 8; 25. Auf diese Literaturstelle wird an dieser Stelle ausdrücklich Bezug genommen. Die Umsetzung erfolgt in hoher Ausbeute zu einem Produkt mit hohem Reinheitsgrad. In gleicher Weise lassen sich, durch unterstöchiometrischen Einsatz von NH₃ und/oder Verbindungen der Formeln III und/oder IV, die analogen Poly-asparaginsäurederivat-co-succinimid-Verbindungen der Formel VIII (n = 1) herstellen.

Zur Herstellung von reinem Poly-(hydroxyalkyl)-L-glutamin muß ein anderes, umständlicheres Verfahren angewendet werden, wie es in der US-PS 4 356 166 beschrieben ist. Dabei wird zunächst die y-ständige COOH-Gruppe der L-Glutaminsäure durch Veresterung mit Benzylalkohol geschützt. Anschließend wird dieses y-Benzyl-Glutamat mit Phosgen zu einem N-Carboxyanhydrid umgesetzt, welches dann nach Zugabe von Triethylamin in einem inerten Lösungsmittel polymerisiert, wobei man Poly-γ-(benzyl)-L-glutamat erhält. Die Abspaltung der Schutzgruppe erfolgt entweder durch Zugabe von einem HCI/HBr-Gemisch zur freien Poly-a-L-Glutaminsäure oder aber in Gegenwart von Hydroxyalkylaminen zu den analogen Poly-a-(hydroxyalkyl)-L-glutaminen. Eine allgemeine Arbeitsvorschrift zur Herstellung von Poly-a-(hydroxypropyl)-L-glutamin findet sich in der US-PS 4 356 166, auf die an dieser Stelle ausdrücklich Bezug genommen wird. In gleicher Weise lassen sich auch durch Einsatz von NH₃ und/oder Verbindungen der Formeln III und/oder IV die analogen Verbindungen der Formel VII (n = 2) herstellen

Gegenüber der aufwendigen Darstellung von reiner Polyglutaminsäure und deren Derivate kann man Glutaminsäure bis zu hohen Anteilen bei der einfachen Kondensation von Asparaginsäure mittels Phosphorsäure zu Polyanhydroasparaginsäure-Co-glutaminsäure mit einbauen.

Die Polyaminosäureamid-co-anhydrodicarbonsäuren der Formel VIII (R'= HN-(CH₂)ₘ-OH) können nun, sofern erforderlich, im folgenden Reaktionsschritt mit einer oder mehreren verschiedenen, biologisch inaktiven Verbindungen der Formel V und/oder VI und/oder VII 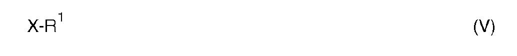 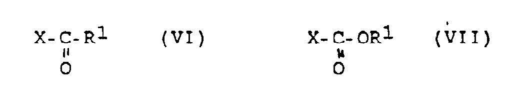 zu weiteren erfindungsgemäßen Polyaminodicarbonsäure-co-AHADS-Derivaten umgesetzt werden. Hierbei steht X für eine Abgangsgruppe, die eine schonende Veresterung der Polymer-Alkoholgruppe ermöglicht. Bevorzugt sind Chlor, Brom, Jod, Imidazolide, Anhydride oder Hydroxyl, insbesondere Chlor.

Die Umsetzung mit den Verbindungen des Formeltyps V, VI oder VII kann sowohl mit einer einzigen solchen Verbindung erfolgen als auch mit beliebigen Kombinationen dieser Verbindungen oder auch mit Verbindungen, die unterschiedliche, z.B. in der Art ihrer Verzweigung, insbesondere in ihrer Kettenlänge verschiedene Reste R^{l} haben.

Die letztgenannte polymeranaloge Alkylierung bzw. Acylierung wird nach bekannten Verfahren der organischen Chemie durchgeführt. Sie verläuft selektiv an der Hydroxylfunktion (Formel VIII, R' = HN-(CH₂)ₘ-OH) zu Ethern, Estern bzw. Carbonaten, ohne weitere Funktionen am Ausgangspolymeren anzugreifen. Besonders eignet sich die Einhorn-Variante der Schotten-Baumann-Acylierung in Gegenwart von Pyridin. Dabei werden unter schonenden Bedingungen sehr hohe Derivatisierungsgrade (größer 70 %) erzielt.

Das Molekulargewicht der erfindungsgemäßen Polymeren beträgt 200 bis 100.000, bevorzugt 3.000 bis 70.000.

Verbindungen des Formeltyps V sind käuflich oder, sofern nicht, auf einfache Weise nach literaturbekannten Verfahren zu synthetisieren.

Die Chlorameisensäureester (Formel VII) erhält man durch Umsetzung von Phosgen mit den entsprechenden biologisch inaktiven, physiologisch unbedenklichen, aromatischen, araliphatischen, aliphatischen oder cycloaliphatischen, insbesondere unverzweigten Alkoholen. Besonders bevorzugt werden solche Alkohole eingesetzt, die eine geradzahlige Kohlenstoffatomanzahl aufweisen. Auf diese Weise erhält man auch die chlorformylierten Steroide. Prinzipiell sind somit alle biologisch inaktiven Steroide zugänglich, die reaktive Hydroxylgruppen aufweisen. Beispielsweise seien hier genannt: Cholesterol, Cholestanol, Coprostanol; Ergosterol, Sitosterol oder Stigmasterol.

Die ebenfalls einsetzbaren Säurechloride (Formel Vl) erhält man beispielsweise aus den entsprechenden Carbonsäuren durch Umsetzung mit Phosphortrichlorid, Phosphorpentachlorid, Oxalylchlorid oder Thionylchlorid.

Verbindungen des Formeltyps V, VI oder VII, in denen eine Alkylkette durch eine Oxycarbonyl- oder Carbonyloxy-Gruppe unterbrochen ist, werden beispielsweise durch Umsetzung von cyclischen Dicarbonsäureanhydriden mit Alkoholen hergestellt. Die auf diese Weise erhaltenen Dicarbonsäuremonoester werden dann analog zu den oben beschriebenen Carbonsäuren, z.B. mit Oxalylchlorid zu den entsprechenden Säurechloriden umgesetzt.

Die Hydrophobie der Polyaminodicarbonsäure-co-AHADS-Derivate- und damit die Verweildauer eines daraus hergestellten Implantats im Organismus - läßt sich sowohl über die Anzahl der C-Atome im Alkylierungsbzw. Acylierungsagens als auch über den Substitutionsgrad sowie den Gehalt an ungeöffnetem AHADS in weiten Grenzen einstellen.

Eine exakte Angabe der Relation Kettenlänge/AHADS-Gehalt/Abbauzeit in vitro/vivo ist jedoch nur schwer möglich, da die Abbauzeit außer von der Kettenlänge und dem AHADS-Gehalt noch von einer Vielzahl anderer Parameter abhängig ist; beispielsweise von der Partikelgröße und -verteilung, der Herstellungsmethode z.B. für Microspheres, der Porosität der Micospheres, der Temperatur oder dem Abbaumedium. Durch einfache Routineversuche im Rahmen der Offenbarung ist es dem Fachmann jedoch möglich, die gewünschte Abbauzeit zu erreichen.

Der Substitutionsgrad in den Verbindungen VIII (R'= HN-(CH₂)ₘ-OH) kann über die Stöchiometrie der bei derAlkylierungs- bzw. Acylierungsreaktion eingesetzten Substanzen verändert werden, sollte vorzugsweise jedoch im Rahmen der maximalen Ausbeute (größer 70 %) gehalten werden, d.h. ein möglichst großer Prozentsatz der substituierbaren OH-Gruppen am Polymergerüst (Formel VIII; R'= HN-(CH₂)ₘ-OH) sollte verestert werden. Ist ein niedrigerer Substitutionsgrad erwünscht, so erniedrigt man entsprechend die Konzentration des Alkylierungs- bzw Acylierungsagens in Bezug auf das Polymere.

Beim Abbau dieser Polyamide in vivo werden a) die Imidringe in der Polymerhauptkette gespalten und b) die reaktiven Seitegruppen in den Polyamiden wieder hydrolysiert und es entstehen die entsprechenden biologisch inaktiven Carbonsäuren bzw. Alkohole und Poly-(hydroxyalkyl)-Aminodicarbonsäure und, sofern zwecks besserer Wasserlöslichkeit vorgesehen, in geringem Maße Oligoaminodicarbonsäuresequenzen. Dieser Abbau unter physiologischen Bedingungen soll idealerweise ausschließlich körpereigene bzw. in ihrer hohen Bioverträglichkeit bekannte Fragmente erzeugen, die in natürlichen Stoffwechselwegen metabolisiert oder auf Grund ihrer Wasserlöslichkeit durch die Nieren ausgeschieden werden. Zu den bioverträglichen Carbonsäuren bzw. Alkoholen zählen solche mit 6 - 22 C-Atomen im Alkylteil, insbesondere solche mit geradzahliger Anzahl von Kohlenstoffatomen.

Das Vorliegen von a- und ß-Peptidbindungen in D- und L-Form in den eingesetzten a,ß-Poly-DL-Aminodicarbon- säureamid-co-AHADS-Derivaten verhindert die Ausbildung organisierter Strukturen (z.B. Faltblattoder Helixbereiche) im Polymeren, die den Bioabbau in nicht vorhersehbarer Weise beeinflussen.

Einige der erfindungsgemäßen Polyamide sind thermoplastisch und eignen sich daher zur Herstellung von Wirkstoffdepotformen nach verschiedenen Methoden, wie z.B. durch Kompression, Extrusion, Fällung, Versprühung, etc.

Aus den erfindungsgemäßen Polyamiden können nach bekannten Methoden implantierbare Partikel, insbesondere Mikrokapseln und Mikrosphären sowie durch Kompaktierung makroskopische Formkörper beliebiger Geometrie, insbesondere Tabletten und Stäbchen hergestellt werden.

Die ausgezeichnete Löslichkeit der erfindungsgemäßen Polymere in vielen organischen Lösungsmitteln ermöglicht auch die Bildung von Mikrokugeln durch Vertropfen aus einem Lösungsmittel mit hohem Schmelzpunkt in ein kondensiertes kaltes Gas, z.B. flüssigen Stickstoff, wobei durch das Leidenfrostsche Phänomen absolut runde Partikel entstehen. Das hochschmelzende und mit Wasser mischbare Lösungsmittel wird durch Überführung der Mikrokugeln in Wasser herausgelöst und das Polymere dabei ausgefällt, wobei die Kugelgestalt der Polyamid Mikrospheres erhalten bleibt.

Besitzt das verwendete organische Lösungsmittel neben einem hohen Schmelzpunkt zugleich einen niedrigen Siedepunkt, so läßt sich dieses Vertropfungsverfahren weiter vereinfachen, indem das Lösungsmittel, z. B. tert. Butanol, direkt mittels Gefriertrocknung der durch Eintropfen in flüssigen Stickstoff gewonnenen Mikrokugeln schonend und ohne Wirkstoffverluste entfernt werden kann.

Besonders vorteilhaft wirkt sich die Löslichkeit der erfindungsgemäßen Polyamide in vielen auch physiologisch verträglichen Lösungsmitteln, z. B. Alkoholen, bei der Verarbeitung zu Mikrospheres mittels Sprühtrocknung aus. So kann auf den Einsatz von toxikologisch bedenklichen halogenierten Kohlenwasserstoffen, wie sie für die Sprühtrocknung von biodegradablen Polyestern erforderlich sind, bei den erfindungsgemäßen Polyamiden verzichtet werden. Darüber hinaus erlaubt ihre Löslichkeit auch in Alkohol/Wasser-Gemischen die Herstellung von monolithischen, wirkstoffhaltigen Mikrokugeln, da hierbei Polymer und Wirkstoff aus molekulardisperser Form versprüht werden können.

Die erfindungsgemäßen Polyamide können auch als Gemische und in Abmischungen mit anderen bioabbaubaren und/oder bioverträglichen Polymeren (z.B. ®Pluronic F68, PHEA, Dextrane, Polyethylenglykole, Hydroxyethylstärke und andere abbaubare oder ausscheidbare Polysaccharide) oder physiologisch unbedenklichen Hilfsstoffen (z. B. Polymerweichmacher) eingesetzt werden.

Abbauversuche in vitro mit den erfindungsgemäßen Polyamiden haben gezeigt, daß die Abbaurate sowohl über die funktionellen Seitengruppen als auch über die Anzahl der AHADS-Einheiten kontrolliert werden kann.

In den folgenden Beispielen wird die Erfindung im einzelnen beschrieben. Prozentangaben beziehen sich auf das Gewicht, sofern nicht anders angegeben.

### Beispiel 1

### Herstellung von Polyanhydroasparginsäure-co-α,ß-(hydroxyethyl)-D,L-aspartamid (70:30)

10 g (103 mmol) Polyanhydroasparaginsäure werden in etwa 40 ml DMF gegebenenfalls unter gelindem Erwärmen gelöst. Zu dieser Lösung werden 1,83 ml (30 mmol) frisch destilliertes 2-Aminoethanol zugetropft und bei Raumtemperatur über Nacht gerührt. Der Reaktionsansatz wird in Butanol gefällt und mit getrocknetem Aceton mehrfach gewaschen. Die Trocknung erfolgt im Vakuum bei erhöhter Temperatur. Das weiße, wasserlösliche Produkt entsteht zu annähernd 100 % und wird NMR-spektroskopisch auf Rückstände von DMF und Butanol geprüft. Das eingesetzte molare Verhältnis von Polyanhydroasparaginsäure zu Aminoethanol entspricht etwa der Copolymerenzusammensetzung.

### Beispiel 2

### Herstellung von 4-Chlor-4-oxobuttersäure-n-butylester

Bernsteinsäuremonobutylester wird mit überschüssigem Thionylchlorid und einem Tropfen DMF versetzt. Die Reaktion springt unter Gasentwicklung an Man läßt den Ansatz über Nacht unter Feuchtigkeitsausschluß rühren und destilliert anschließend das überschüssige Thionylchlorid bei Normaldruck ab. Das zurückbleibende Rohprodukt wird bei 0,05 mbar fraktioniert destilliert und das Reinprodukt bei ca. 70°C gewonnen. Bei der IR-spektroskopischen Charakterisierung weist das Produkt Banden bei 1800 cm-⁷ (Säurechlorid) und 1740 cm-' (Ester) von gleicher Intensität auf.

### Beispiel 3

### Herstellung von Polyanhydroasparaginsäure-co-α,ß-(butyloxycarbonylpropionyloxyethyl)-D,L-aspartamid (70:30)

6 g Polyanhydroasparaginsäure-co-a,ß-(hydroxyethyl)-D,L-aspartamid (₌ 16 mmol Hydroxyethylgruppen), dargestellt wie in Beispiel 1 beschrieben, werden in 100 ml trockenem N,N-Dimethylformamid (DMF) gelöst. Nach Zugabe von 4 g (50 mmol) Pyridin wird auf 0°C gekühlt und unter Rühren innerhalb 15 Minuten 4,8 g (25 mmol) 4-Chlor-4-oxobuttersäure-n-butylester (Darstellung Bsp. 2) zugegeben. Der Ansatz wird über Nacht gerührt und in 0,51 Ether gefällt. Das ausgefallene Produkt wird abgesaugt, mit Ether, Aceton, Wasser, Aceton und Ether gewaschen. Man erhält etwa 8 g eines weißen Polymeren mit einem Substitutionsgrad von annähernd 100 % (NMR-spektroskopisch überprüfbar). Das entstandene Polymer ist z. B. in Acetonitril mit einer Spur DMSO, in DMSO oder DMF löslich.

### Beispiel 4

### Herstellung von Polyanhydroasparaginsäure-co-α,ß-(nonylcarbonyloxyethyl)-D.L-aspartamid (50:50)

6 g eines Polyanhydroasparaginsäure-co-a,ß-(hydroxyethyl)-D,L-aspartamids (50:50) (≙ 24 mmol Hydroxyethylgruppen), das analog Beispiel 1 aus Polyanhydroasparaginsäure (MG = 14000) und 2-Aminoethanol (molares Verhältnis 2: 1) hergestellt wurde, werden in 100 ml trockenem DMF gelöst, mit 8 g (100 mmol) trockenem Pyridin versetzt und auf 0°C gekühlt. Es werden langsam 9,6 g destilliertes Decansäurechlorid zugetropft und analog Beispiel 3 weitergearbeitet. Man erhält etwa 8 g eines weißen, vollständig substituierten Polymers (NMR-Kontrolle), das z. B. in Dichlormethan und THF mit jeweils einer Spur DMSO oder in Methanol/Dichlormethan-Gemischen löslich ist.

### Beispiel 5

### Herstellung von Polyanhydroasparaginsäure-co-α,ß-(nonylcarbonyloxyethyl)-D,L-aspartamid verschiedener Copolymerenzusammensetzung und unterschiedlichen Molekulargewichtes.

Analog Beispiel 1 wurden verschiedene Polyanhydroasparaginsäure-co-a,ß-(hydroxyethyl)-D,L-aspartamide u. a. der Zusammensetzung 70:30, 50:50 und 30:70 aus Polyanhydroasparaginsäuren unterschiedlichen Molekulargewichtes (MG = 7000: ca. 13000; 30000) hergestellt und mit Decansäurechlorid, wie in Beispiel 4 beschrieben zu den entsprechenden Polyanhydroasparaginsäure-co-α,ß-(nonylcarbonyloxyethyl)-D,L-aspartamiden umgesetzt.
a) - Polyanhydroasparaginsäure-co-α,ß-(nonylcarbonyloxyethyl)-D,L-aspartamid (70:30) aus Polyanhydroasparaginsäure (MG = 7000); charakterisiert durch NMR
b) - Polyanhydroasparaginsäure-co-a,ß-(nonylcarbonyloxyethyl)-D,L-asoariamid (70:30) aus Polyanhydroasparaginsäure (MG = 14000); charakterisiert durch NMR
c) - Polyanhydroasparaginsäure-co-α,ß-(nonylcarbonyloxyethyl)-D.L-aspartamid (70:30) aus Polyanhydroasparaginsäure (MG = 30000); charakterisiert durch NMR
d) - Polyanhydroasparaginsäure-co-α,ß-(nonylcarbonyloxyethyl)-D,L-aspartamid (30:70) aus Polyanhydroasparaginsäure (MG 12000); charakterisiert durch NMR

Mikropartikel aus diesen Produkten unterscheiden sich deutlich in Löslichkeit Konsistenz. Abbauverhalten und Wirkstoffreisetzung (Beispiele 12, 13)

### Beispiel 6

### Herstellung von Polyanhydroasparaginsäure-co-α,ß-(octyloxycarbonyloxyethyl)-D,L-aspartamid (70:30)

6 g Polyanhydroasparaginsäure-co-a,ß-(hydroxyethyl)-D,L-aspartamid (70:30) (= 16 mmol Hydroxyethylgruppen), dargestellt wie in Beispiel 1 beschrieben aus Polyanhydroasparaginsäure (MG =3₇000) und Aminoethanol, werden analog Beispiel 3 mit 4,8 g (25 mmol) Octylchloroformiat umgesetzt sowie entsprechend aufgearbeitet. Man erhält etwa 8 g eines weißen, vollständig substituierten Polymers, das in THF oder Methanol/Dichlormethanol-Gemischen löslich ist.

### Beispiel 8

### Herstellung von Polyanhydroasparaginsäure-co-a,ß-(oleyloxyethyl)-D,L-aspartamid (10:90)

6 g Polyanhydroasparaginsäure-co-a,ß-(hydroxyethyl)-D,L-aspartamid (10:90) (≙ 40 mmol Hydroxyethylgruppen), dargestellt analog Beispiel 1 mit einem molaren Verhältnis von Polyanhydroasparaginsäure zu 2-Aminoethanol wie 1:9, werden mit 20 g destilliertem Ölsäurechlorid analog Beispiel 3 umgesetzt. Die heterogene Reaktionsmischung wird durch Zugabe von Dichlormethan homogen. Es wird zweifach in Methanol, das auf -20°C gekühlt ist, gefällt. Das gelblich gefärbte Polymer ist thermoplastisch.

### Beispiel 9

### Herstellung von stäbchenförmigen Implantaten ("rods")

Ein inniges Gemisch aus pulverförmigen, thermoplastisch verarbeitbaren Polymeren (Substanz aus Beispiel 8), Additiven und Wirkstoff(en) wird in einer geeigneten Vorrichtung, z. B. einem Extruder für Thermoplasten, über den Erweichungspunkt erhitzt, wobei eine verformbare Masse entsteht. Durch Kneten werden Additive und Wirkstoff(e) im erweichten Polymer homogen dispergiert und die erhaltene Polymer/WirkstoffSuspension durch eine Düse geeigneten Durchmessers (>0,5 mm) gepreßt. Beim Abkühlen erstarrt der Strang der extrudierten Polymer/Wirkstoff-Suspension zu einem festen stäbchenförmigen Aggregat, dessen Wirkstoffgehalt von seiner Länge und seinem Durchmesser bestimmt ist.

### Beispiel 10

a) Herstellung von Microspheres
   40 mg Polyanhydroasparaginsäure-co-a,ß-(nonylcarbonyloxyethyl)-D,L-aspartamid (50:50) aus Beispiel 4 werden in 1 ml Methylenchlorid/Methanol (Volumenanteil 50/1) gelöst. Die Lösung wird mit 10 mg Buserelin versetzt, das mit Ultraschall dispergiert wird. Die Dispersion wird unter Rühren (800 Upm) in ein Becherglas mit 60 ml 0, 1 Gew.-%iger, wäßriger Polyvinylalkohol-Lösung (®Mowiol28-99) eingebracht, die mit 0,3 ml Methylenchlorid/ Methanol (50/1) gesättigt ist.
   Nach 5 Minuten wird der Inhalt in ein Becherglas mit 200 ml Wasser gegeben und 30 Minuten gerührt (200 Upm). Das überstehende Wasser wird abdekantiert und die Microspheres werden lyophilisiert (Durchmesser nach Lyophilisation: 20-90 pm)
b) Herstellung von Microspheres
   80 mg Polyanhydroasparaginsäure-co-a,ß-(octylcarbonyloxyethyl)-D,L-aspartamid (70:30) aus Beispiel 6 werden bei 50°C in 1 ml Dimethylsulfoxid gelöst und mit 20 mg Hydroxypropylcellulose (®Klucel M.) versetzt. Die Lösung der beiden Polymeren wird mit einer Kanüle (Einwegspritze, Kanülendurchmesser außen 0,6 mm) in eine Vorlage von flüssigem Stickstoff (100 ml) eingetropft.
   Die entstandenen Microspheres werden in 200 ml Wasser überführt und 2 Stunden von restlichem Lösungsmittel extrahiert. Überschüssiges Wasser wird abdekaniert und die Microspheres werden lyophilisiert (Durchmesser nach Lyophilisation: 1-2 gm).
c) 3,76 g Polyamid nach Beispiel 5 c) wird in 195 ml Dichlormethan (0,25 % DMSO) unter Erwärmen gelöst und mit einer Lösung von 240 mg Buserelin in 5 ml Wasser vereinigt. Das Gemisch wird in einem Sprühtrockner zu Mikrokugeln versprüht.
d) 3,76 g Polyamid nach Beispiel 5 d) wird in 195 ml Dichlormethan (0,25 % DMSO) unter Erwärmen gelöst und mit einer Lösung von 240 mg Buserelin in 5 ml Wasser vereinigt. Das Gemisch wird in einem Sprühtrockner zu Mikrokugeln versprüht.
e) 3,76 g Polyamid nach Beispiel 6 wird in 195 ml Dichlormethan (0,25 % DMSO) unter Erwärmen gelöst und mit einer Lösung von 240 mg Buserelin in 5 ml Wasser vereinigt. Das Gemisch wird in einem Sprühtrockner zu Mikrokugeln versprüht.
f) 3,76 g Polyamid nach Beispiel 4 wird in 195 ml Dichlormethan (0,25 % DMSO) unter Erwärmen gelöst und mit einer Lösung von 240 mg Buserelin in 5 ml Wasser vereinigt. Das Gemisch wird in einem Sprühtrockner zu Mikrokugeln versprüht.
g) 3,76 g Polylactidglycolid (50:50) (PLGA 50:50) (Boehringer Ingelheim) wird in 195 ml Dichlormethan (0,25 % DMSO) unter Erwärmen gelöst und mit einer Lösung von 240 mg Buserelin in 5 ml Wasser vereinigt. Das Gemisch wird in einem Sprühtrockner zu Mikrokugeln versprüht.
h) 3,76 g Polyamid nach Beispiel 5 d) wird in 195 ml Tetrahydrofuran (0,25 % DMSO) unter Erwärmen gelöst und mit einer Lösung von 240 mg Buserelin in 5 ml Wasser vereinigt. Die Lösung wird in einem Sprühtrockner zu Mikrokugeln versprüht.

### Beispiel 11

### Wasserresorption

Die Wasserresorption der Polyamide nach 74 h Lagerung bei 92 % relativer Luftfeuchte liegt für Polyanhydroasparaginsäure-co-a,ß-(nonylcarbonyloxyethyl)-D,L-aspartamid (70:30) aus Polyanhydroasparaginsäure (MG = 30000) nach Beispiel 5 c) und für Polyanhydroasparaginsäure-co-a,ß-(octyloxycarbonyloxyethyl)-D,L-aspartamid (70:30) aus Polyanhydroasparaginsäure (MG = 37000) nach Beispiel 6 bei 8-10 Gew.-%.

### Beispiel 12

### Polymerabbau

4 Proben von jeweils 120 mg Polymer werden in je 30 ml einer Phosphat-Pufferlösung aus 2,05 mmol Na₂HP0₄, 4,5 mmol NaH₂P0₄ und 7,8 mmol NaN₃ (pH 7,4) in verschlossenen Glasflaschen (50 ml) bei 37°C inkubiert. Die Gewichtsabnahme der Polymerproben wurde gemessen, indem die Pufferlösung mit inkubiertem Polymer über eine tarierte Glasfritte filtriert, der Rückstand 24 h im Vakuum über Phosphorpentoxid getrocknet und die Gewichtsabnahme bestimmt wird.

Beispielreihe mit Polyanhydroasparaginsäure-co-a,ß-(nonylcarbonyloxyethyl)-D,L-aspartamiden verschiedener Copolymerenzusammensetzung (x:y) und unterschiedlichen Molekulargewichten (aus Beispielen 4 und 5).

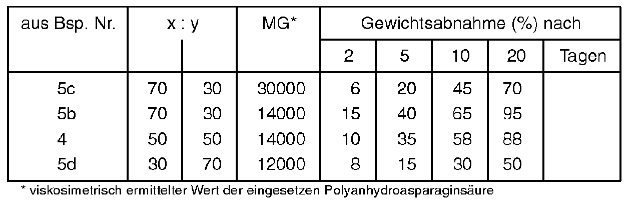

### Beispiel 13

### Wirkstofffreigabe (Release von Buserelin) nach 24 h (Initial burst)

In 10 ml-Probengläschen wurden 20 mg Mikropartikel mit einer 6 %igen Beladung von Buserelin eingewogen und mit 5 ml lecithinhaltiger Pufferlösung (2,91 g Na₂HPO₄, 0,54 g NaH₂PO₄, 0,1 g NaN₃, 6,33 g NaCl, 2,52 g NaHCO₃ und 10 g Eilecithin auf 1 I H₂O) versetzt. Die Proben wurden mit Magnetrührkernen so gerührt, daß die Mikropartikel in Suspension gehalten wurden und sich keine Partikel auf der Oberfläche bzw den Gefäßwänden ansammeln sowie bei Mikropartikeln klebriger Konsistenz keine größeren Aggregate entstehen konnten. Nach 24 Stunden wurden die Proben filtriert und im Filtrat der Buserelingehalt mittels HPLC bestimmt.

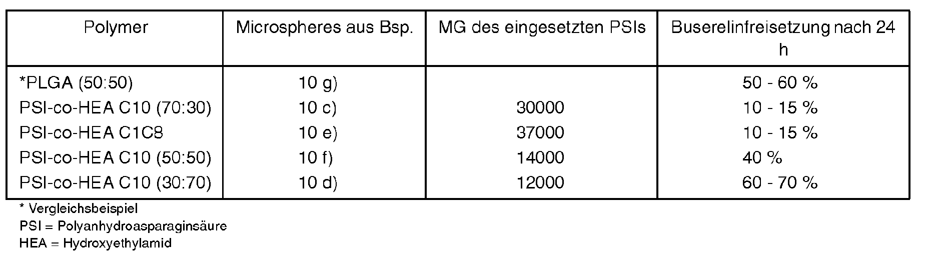

## Patentansprüche (Patentansprüche für folgende(n) Vertragsstaat(en) : BE, DE, DK, FR, GB, IT, NL)

1. Polyaminodicarbonsäure-co-anhydroaminodicarbonsäure-Derivate der Formel 1, 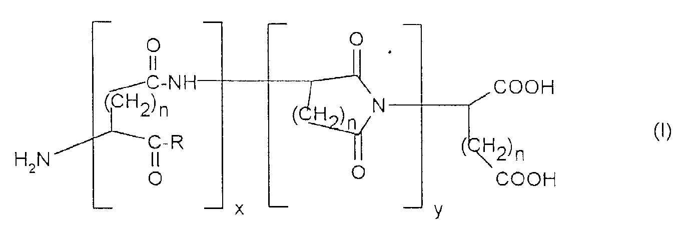 in der
n oder2
x 1 bis 500
y 1 bis 500 ist, wobei
x + y 2 bis 1000 ist und
R O-R¹ oder NH-R² bedeutet, worin
R² H, (CH₂)ₘ-OR¹, (CH₂)ₘ-O-C(O)-R¹ oder (CH₂)ₘ-O-C(O)-OR¹ bedeutet und
m 2 bis 6 ist und
R¹ H, Aryl, Aralkyl, Arylalkenyl, Alkyl oder C₃-C₈-Cycloalkyl oder ein biologisch inaktiver Steroidalkohol- oder ein Aminosäure-Rest bedeutet, wobei Aryl unsubstituiert ist oder substituiert ist mit C₁-C₄-Alkyl, C₂-C₄-Alkenyl, C₁-C₄-Alkylcarbonyloxy, C₁-C₄-Alkoxycarbonyl, C₁-C₄-Alkoxy oder Hydroxy
wobei die für R^{l} genannten Alkylreste 1 - 22 C-Atome und die Alkenylreste 2 - 22 C-Atome aufweisen, die nicht unterbrochen oder durch eine Carbonyloxy- oder Oxycarbonylgruppe unterbrochen sind, wobei die in eckige Klammern gesetzten Wiederholungseinheiten statistisch und/oder in Blöcken im Polymeren verteilt sind, wobei sowohl die mit x als auch die mit y gekennzeichneten Wiederholungseinheiten identisch sind, wobei die Aminosäuren a-und/oder ß- verknüpft sind, wobei eine Verbindung der Formel bei der n 1, m 2, R -NHR², R² -(CH₂)ₘ-OR¹, R¹ -H und x gleich y ist, ausgeschlossen ist und wobei eine Verbindung der Formel bei der n 1 und R -OH bedeuten und x und y die in Formel I genannte Bedeutung haben, ausgeschlossen ist.

2. Poly-aminodicarbonsäure-co-anhydroaminodicarbonsäure-Derivate der Formel nach Anspruch 1, worin
R NH-R² bedeutet und
m 2 ist und
R¹ H, Aryl, Aralkyl, Alkyl oder C₅-C₈-Cycloalkyl bedeutet, wobei die Alkylreste 1 - 22 C-Atome aufweisen.

3. Poly-aminodicarbonsäure-co-anhydroaminodicarbonsäure-Derivate der Formel nach Anspruch 1, worin
R O-R¹ bedeutet und
R¹ Aryl, Aralkyl, Alkyl oder C₅-C₆-Cycloalkyl bedeutet, wobei die Alkylreste 1 - 22 C-Atome aufweisen.

4. Verfahren zur Herstellung von Polyaminodicarbonsäure-coanhydroaminodicarbonsäure-Derivaten der Formel I, dadurch gekennzeichnet, daß man eine Polyanhydroaminodicarbonsäure der Formel II 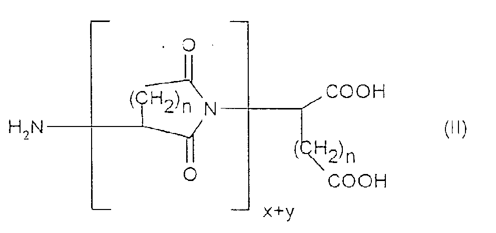 worin n, x und y die in Anspruch 1 angegebenen Bedeutungen haben, partiell umsetzt mit NH₃ oder einer Verbindung der Formeln III oder IV 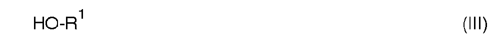 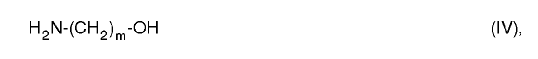 worin
R¹ und m die in Anspruch 1 angegebenen Bedeutungen haben und gegebenenfalls anschließend in einer polymeranalogen Umsetzung das erhaltene Produkt mit einer oder mehreren Verbindungen der Formeln V, VI oder Vll 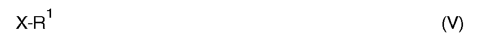 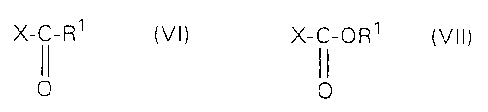 worin R¹ wie in Anspruch 1 definiert ist und X eine Abgangsgruppe ist, umsetzt

5. Verwendung eines Polyamids nach Anspruch 1, 2 oder 3 zum Herstellung von biologisch abbaubaren Wirkstoffdepotzubereitungen mit kontrollierter Wirkstoffabgabe.

6. Verwendung eines Polyamids nach Anspruch 1, 2 oder 3 zum Einkapseln von biologisch aktiven Subtanzen.

7. Verwendung eines Polyalids nach Anspruch 1, 2 oder 3 Abmischungen mit anderen bioabbaubaren und/oder bioverträglichen Polyamiden oder physiologisch unbedenklichen Hilfsstoffen zur Herstellung von biologisch abbaubaren Wirkstoffdepotzubereitungen mit kontrollierter Wirkstoffabgabe.

## Patentansprüche (Patentansprüche für folgende(n) Vertragsstaat(en) : ES)

1. Verfahren zur Herstellung von Polyaminodicarbonsäure-coanhydroaminodicarbonsäure-Derivaten der Formel 1, 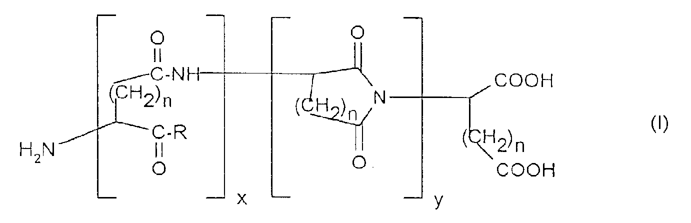 in der
n 1 oder 2,
x 1 bis 500,
y 1 bis 500 ist, wobei x + y 2 bis 1000 ist und
R O-R¹ oder NH-R² bedeutet, worin
R² H, (CH₂)ₘ-OR¹, (CH₂)ₘ-O-C(O)-R¹ oder (CH₂)ₘ-O-C(O)-OR¹ bedeutet und
m 2 bis 6 ist und
R¹ H, Aryl, Aralkyl, Arylalkenyl, Alkyl oder C₃-C₈-Cycloalkyl oder ein biologisch inaktiver Steroidalkohol- oder ein Aminosäure-Rest bedeutet, wobei Aryl unsubstituiert ist oder substituiert ist mit C₁-C₄-Alkyl, C₂-C₄-Alkenyl, Cᵢ-C₄-Alkylcarbonyloxy, C₁-C₄-Alkoxycarbonyl, C₁-C₄-Alkoxy oder Hydroxy,
wobei die für R¹ genannten Alkylreste 1 - 22 C-Atome und die Alkenylreste 2 - 22 C-Atome aufweisen, die nicht unterbrochen oder durch eine Carbonyloxy- oder Oxycarbonylgruppe unterbrochen sind, wobei die in eckige Klammern gesetzten Wiederholungseinheiten statistisch und/oder in Blöcken im Polymeren verteilt sind, wobei sowohl die mit x als auch die mit y gekennzeichneten Wiederholungseinheiten identisch sind, wobei die Aminosäuren a-und/oder ß- verknüpft sind, wobei eine Verbindung der Formel I, bei der n 1, m 2, R -NHR², R₂-(CH₂)ₘ-OR¹, R¹ -H und x gleich y ist, ausgeschlossen ist und wobei eine Verbindung der Formel bei der n 1 und R -OH bedeuten und x und y die in Formel I genannte Bedeutung haben, ausgeschlossen ist, dadurch gekennzeichnet, daß man eine Polyanhydroaminodicarbonsäure der Formel II 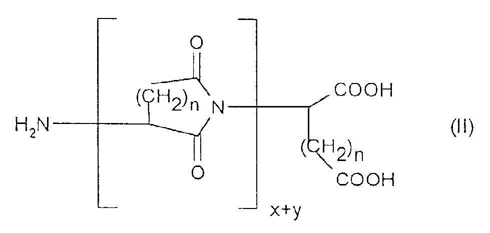 worin n, x und y die in Anspruch 1 angegebenen Bedeutungen haben, partiell umsetzt mit NH₃ oder einer Verbindung der Formeln III oder IV 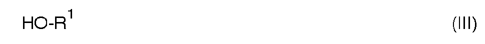 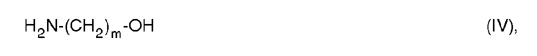 worin R¹ und m die oben angegebenen Bedeutungen haben und gegebenenfalls anschließend in einer polymeranalogen Umsetzung das erhaltene Produkt mit einer oder mehreren Verbindungen der Formeln V, VI oder VII 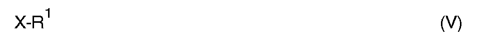 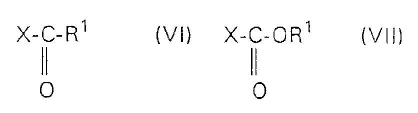 worin R¹ wie oben definiert ist und X eine Abgangsgruppe ist, umsetzt.

2. Verfahren nach Anspruch 1, dadurch gekennzeichnet, daß man Poly-aminodicarbonsäure-coanhydroaminodicarbonsäure-Derivate der Formel I, worin
R NH-R² bedeutet und
m 2 ist und
R¹ H, Aryl, Aralkyl, Alkyl oder C₅C₈-Cycloalkyl bedeutet, wobei die Alkylreste 1 - 22 C-Atome aufweisen, herstellt.

3. Verfahren nach Anspruch 1 oder 2, dadurch gekennzeichnet, daß man Poly-aminodicarbonsäure-coanhydroaminodicarbonsäure-Derivate der Formel I, worin
R O-R^{l} bedeutet und
R^{l} Aryl, Aralkyl, Alkyl oder C₅-C₈-Cycloalkyl bedeutet, wobei die Alkylreste 1 - 22 C-Atome aufweisen,
herstellt.

4. Verwendung eines Polyamids nach Anspruch 1,2 oder 3 zur Herstellung von biologisch abbaubaren Wirkstoffdepotzubereitungen mit kontrollierter Wirkstoffabgabe.

5. Verwendung eines Polyamids nach Anspruch 1, 2 oder 3 zum Einkapseln von biologisch aktiven Substanzen.

6. Verwendung eines Polyamids nach Anspruch 1, 2 oder 3 in Abmischungen mit anderen bioabbaubaren und/oder bioverträglichen Polyamiden oder physiologisch unbedenklichen Hilfsstoffen zur Herstellung von biologisch abbaubaren Wirkstoffdepotzubereitungen mit kontrollierter Wirkstoffabgabe.

## Claims (Claims for the following Contracting State(s) : BE, DE, DK, FR, GB, IT, NL)

1. A polyaminodicarboxylic acid-co-anhydroaminodicarboxylic acid derivative of the formula I 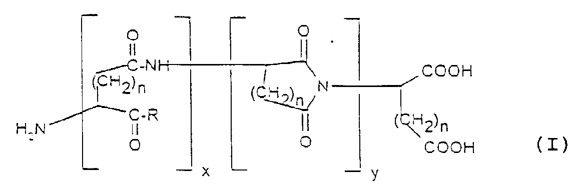 in which
n is 1 or 2,
x is 1 to 500,
y is 1 to 500, where x + y is 2 to 1000 and
R is O-R¹ or NH-R², in which
R² is H, (CH₂)ₘ-OR¹, (CH₂)ₘ-O-C(O)-R¹ or (CH₂)ₘ-O-C(O)-OR¹ and
m is 2 to 6 and
R¹ is H, aryl, aralkyl, arylalkenyl, alkyl or C₃-C₈-cycloalkyl or a biologically inactive steroid alcohol radical or an amino acid residue, where aryl is unsubstituted or substituted by Cₗ-C₄-alkyl, C₂-C₄-alkenyl; Cₗ-C₄-alkylcar- bonyloxy, C₁-C₄-alkoxycarbonyl, C₁-C₄-alkoxy or hydroxyl,
where said alkyl radicals for R¹ have 1 - 22 carbon atoms and the alkenyl radicals have 2 - 22 carbon atoms, which are uninterrupted or interrupted by a carbonyloxy or oxycarbonyl group, where the repeating units in square brackets are distributed randomly and/or in blocks in the polymer, where the repeating units indicated by both x and y are identical, where the amino acids are a- and/or β-linked, where a compound of the formula I in which n is 1, m is 2, R is -NHR², R² is -(CH₂)ₘ-OR¹, R¹ is -H and x is identical to y is excluded and where a compound of the formula I in which n is 1 and R is -OH and x and y have the meaning mentioned in formula I is excluded.

2. A polyaminodicarboxylic acid-co-anhydroaminodicarboxylic acid derivative of the formula I as claimed in claim 1, in which
R is NH-R² and
m is 2 and
R¹ is H, aryl, aralkyl, alkyl or C₅-C₆-cycloalkyl, where the alkyl radicals have 1 - 22 carbon atoms.

3. A polyaminodicarboxylic acid-co-anhydroaminodicarboxylic acid derivative of the formula I as claimed in claim 1, in which
R is O-R¹ and
R¹ is aryl, aralkyl, alkyl or C₅-C₆-cycloalkyl, where the alkyl radicals have 1 - 22 carbon atoms.

4. A process for the preparation of polyaminodicarboxylic acid-co-anhydroaminodicarboxylic acid derivatives of the formula I, which comprises partially reacting a polyanhydroaminodicarboxylic acid of the formula II 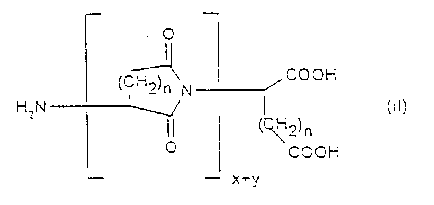 in which n, x and y have the meanings indicated in claim 1, with NH₃ or a compound of the formula III or IV 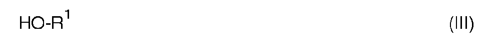 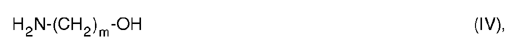 in which
R¹ and m have the meanings indicated in claim 1 and optionally subsequently reacting the product obtained, in a polymer-analogous reaction, with one or more compounds of the formula V, VI or VII 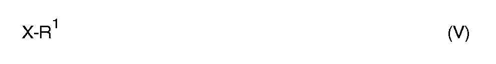 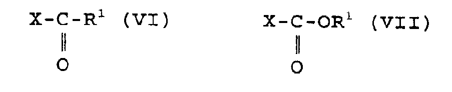 in which R¹ is as defined in claim 1 and X is a leaving group.

5. The use of a polyamide as claimed in claim 1, 2 or 3 for the preparation of biologically degradable active compound depot preparations having controlled delivery of active compound.

6. The use of a polyamide as claimed in claim 1, 2 or 3 for encapsulating biologically active substances.

7. The use of a polyamide as claimed in claim 1, 2 or 3 in mixtures with other biodegradable and/or biotolerable polyamides or physiologically acceptable auxiliaries for the preparation of biologically degradable active compound depot preparations having controlled delivery of active compound.

## Claims (Claims for the following Contracting State(s) : ES)

1. A process for the preparation of a polyaminodicarboxylic acid-co-anhydroaminodicarboxylic acid derivative of the formula I 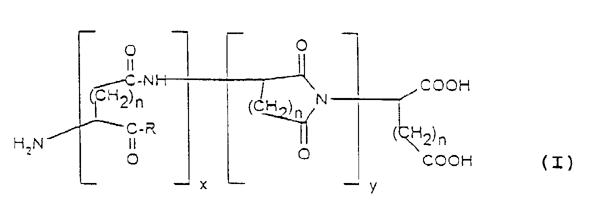 in which
n is 1 or 2,
x is 1 to 500,
y is 1 to 500, where x + y is 2 to 1000 and
R is O-R^{l} or NH-R², in which
R² is H, (CH₂)ₘ-OR¹, (CH₂)ₘ-O-C(O)-R¹ or (CH₂)ₘ-O-C(O)-OR¹ and
m is 2 to 6 and
R¹ is H, aryl, aralkyl, arylalkenyl, alkyl or C₃-C₈-cycloalkyl or a biologically inactive steroid alcohol radical or an amino acid residue, where aryl is unsubstituted or substituted by Cₗ-C₄-alkyl, C₂-C₄-alkenyl; Cₗ-C₄-alkylcar- bonyloxy, C₁-C₄-alkoxycarbonyl, C₁-C₄-alkoxy or hydroxyl,
where said alkyl radicals for R¹ have 1 - 22 carbon atoms and the alkenyl radicals have 2 - 22 carbon atoms, which are uninterrupted or interrupted by a carbonyloxy or oxycarbonyl group, where the repeating units in square brackets are distributed randomly and/or in blocks in the polymer, where the repeating units indicated by both x and y are identical, where the amino acids are a- and/or β-linked, where a compound of the formula I in which n is 1, m is 2, R is -NHR², R² is -(CH₂)ₘ-OR¹, R¹ is -H and x is identical to y is excluded and where a compound of the formula I in which n is 1 and R is -OH and x and y have the meaning mentioned in formula I is excluded, which comprises partially reacting a polyanhydroaminodicarboxylic acid of the formula II 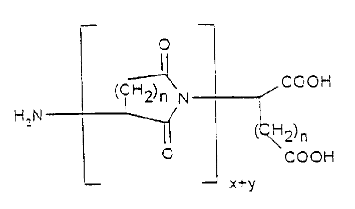 in which n, x and y have the meanings indicated above, with NH₃ or a compound of the formula III or IV 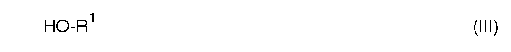 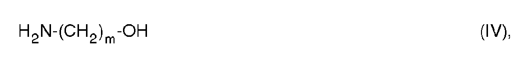 in which R¹ and m have the meanings indicated above and optionally subsequently reacting the product obtained, in a polymer-analogous reaction, with one or more compounds of the formula V, VI or VII 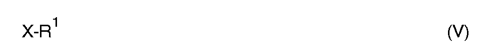 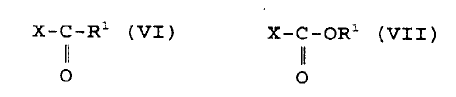 in which R¹ is as defined above and X is a leaving group.

2. The process as claimed in claim 1, wherein a polyaminodicarboxylic acid-co-anhydroaminodicarboxylic acid derivative of the formula I is prepared, in which
R is NH-R² and
m is 2 and
R¹ is H, aryl, aralkyl, alkyl or C₅-C₆-cycloalkyl, where the alkyl radicals have 1 - 22 carbon atoms.

3. The process as claimed in claim 1 or 2, wherein a polyaminodicarboxylic acid-co-anhydroaminodicarboxylic acid derivative of the formula I is prepared, in which
R is O-R¹ and
R¹ is aryl, aralkyl, alkyl or C₅-C₆-cycloalkyl, where the alkyl radicals have 1 - 22 carbon atoms.

4. The use of a polyamide as claimed in claim 1, 2 or 3 for the preparation of biologically degradable active compound depot preparations having controlled delivery of active compound.

5. The use of a polyamide as claimed in claim 1, 2 or 3 for encapsulating biologically active substances.

6. The use of a polyamide as claimed in claim 1, 2 or 3 in mixtures with other biodegradable and/or biotolerable polyamides or physiologically acceptable auxiliaries for the preparation of biologically degradable active compound depot preparations having controlled delivery of active compound.

## Revendications (Revendications pour l'(les) Etat(s) contractant(s) suivant(s) : BE, DE, DK, FR, GB, IT, NL)

1. Copolymères acide aminodicarboxylique/acide anhydroaminodicarboxylique de formule 1 : 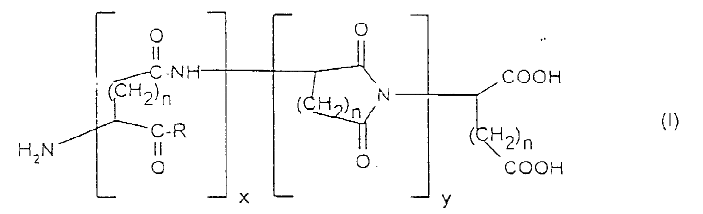 dans laquelle
n vaut 1 ou 2,
x vaut 1 à 500,
y vaut 1 à 500, à la condition que x + y vale de 2 à 1000, et
R représente O-R¹ ou NH-R², où
R² représente H, (CH₂)ₘ-OR¹, (CH₂)ₘ-O-C(O)-R¹ ou (CH₂)ₘ-O-C(O)-OR¹ et
m vaut de 2 à 6 et
R¹ représente H, aryle, aralkyle, arylalcényle, alkyle ou (C₃-C₈)cycloalkyle ou bien un résidu de stérol biologiquement inactif ou un résidu d'acide aminé biologiquement inactif, où le groupe aryle est non substitué ou substitué avec des substituants alkyle en C₁-C₄, alcényle en C₂-C₄, alkyl(C₁-C₄)carbonyloxy, alkoxy(C₁-C₄) carbonyle, alkoxy(C₁-C₄) ou hydroxy, et
le groupe alkyle mentionné à propos de R¹ comporte de 1 à 22 atomes de carbone et le groupe alcényle comporte de 2 à 22 atomes de carbone, qui ne sont pas interrompus ou qui sont interrompus par un groupe carbonyloxy ou oxycarbonyle, et
les motifs répétitifs placés entre crochets sont répartis statistiquement et/ou en bloc dans le polymère, les motifs répétitifs caractérisés aussi bien par x que par y étant identiques, les acides aminés étant liés par la position a ou ß, un composé de formule I dans lequel n est 1, m est 2, R est -NHR², R² est -(CH₂)ₘ-OR¹ R¹ est -H et x est égal à y étant exclu et un composé de formule I, dans lequel n = 1 et R représente -OH et x et y sont tels que définis ci-dessus pour la formule 1 étant exclu.

2. Copolymères acide aminodicarboxylique/acide anhydroaminodicarboxylique de formule 1 selon la revendication 1, caractérisés en ce que
R représente NH-R2 et
m est 2 et
R¹ représente H, aryle, aralkyle, alkyle ou C₅-C₆-cycloalkyle, les groupes alkyle présentant de 1 à 22 atomes de carbone.

3. Copolymères acide aminodicarboxylique/acide anhydroaminodicarboxylique de formule I selon la revendication 1, caractérisés en ce que
R représente O-R¹ et
R¹ représente aryle, aralkyle, alkyle ou C₅-C₆-cycloalkyle, les groupes alkyle présentant de 1 à 22 atomes de carbone.

4. Procédé pour la préparation de copolymères acide aminodicarboxylique/acide anhydroaminodicarboxylique de formule I, caractérisé en ce que l'on fait partiellement réagir un poly(acide anhydroaminodicarboxylique) de formule II: 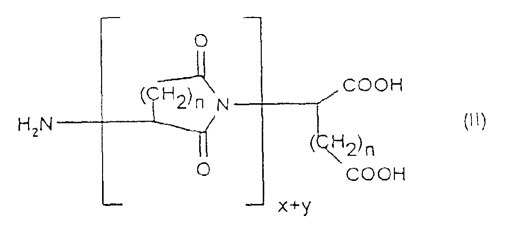 dans laquelle n, x et y sont tels que définis ci-dessus à la revendication 1, avec NH₃ ou un composé de formule III ou IV: 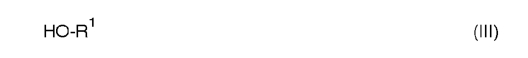 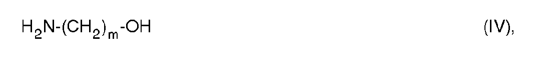 où
R¹ et m ont les significations données ci-dessus et on fait ensuite éventuellement réagir au cours d'une réaction de polymérisation le produit obtenu avec un ou plusieurs composés de formule V, VI ou VII: 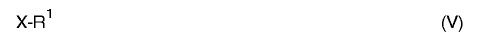 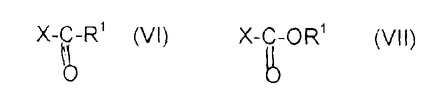 où R¹ est tel que défini ci-dessus et X est un groupe partant.

5. Utilisation d'un polyamide selon la revendication 1, 2 ou 3, pour la préparation de formulations retard, biologiquement dégradables, à libération contrôlée du principe actif.

6. Utilisation d'un polyamide selon la revendication 1, 2 ou 3, pour l'encapsulage de substances biologiquement actives.

7. Utilisation d'un polyamide selon la revendication 1, 2 ou 3, en mélange avec d'autres polyamides biodégradables et/ou biocompatibles, ou des adjuvants physiologiquement acceptables pour la préparation de formes retard, biologiquement dégradables, à libération contrôlée du principe actif.

## Revendications (Revendications pour l'(les) Etat(s) contractant(s) suivant(s) : ES)

1. Procédé pour la préparation de copolymères acide aminodicarboxylique/acide/acide anhydroaminodicarboxylique de formule I : 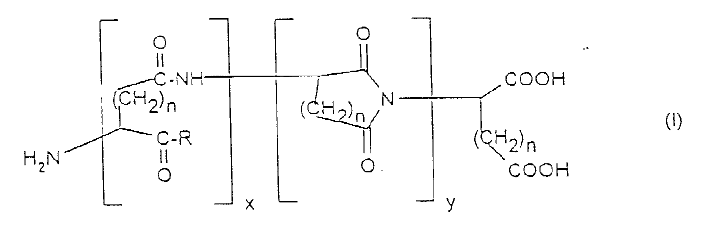 dans laquelle
n vaut 1 ou 2,
x vaut 1 à 500,
y vaut 1 à 500, à la condition que x + y vale de 2 à 1000, et
R représente O-R¹ ou NH-R², où
R² représente H, (CH₂)ₘ-OR¹, (CH₂)ₘ-O-C(O)-R¹ ou (CH₂)ₘ-O-C(O)-OR¹ et
m vaut de 2 à 6 et
R¹ représente H, aryle, aralkyle, arylalcényle, alkyle ou (C₃-Cₛ)cycloalkyle ou bien un résidu de stérol biologiquement inactif ou un résidu d'acide aminé biologiquement inactif, où le groupe aryle est non substitué ou substitué avec des substituants alkyle en C₁-C₄, alcényle en C₂-C₄, alkyl(Cₗ-C₄)carbonyloxy, alkoxy(C₁-C₄) carbonyle, alkoxy(C₁-C₄) ou hydroxy, et
le groupe alkyle mentionné à propos de R¹ comporte de 1 à 22 atomes de carbone et le groupe alcényle comporte de 2 à 22 atomes de carbone, qui ne sont pas interrompus ou qui sont interrompus par un groupe carbonyloxy ou oxycarbonyle, et
les motifs répétitifs placés entre crochets sont répartis statistiquement et/ou en bloc dans le polymère, les motifs répétitifs caractérisés aussi bien par x que par y étant identiques, les acides aminés étant liés par la position a ou ß, un composé de formule I dans lequel n est 1, m est 2, R est -NHR², R² est -(CH₂)ₘ-OR¹ R¹ est -H et x est égal à y étant exclu et un composé de formule I, dans lequel n = 1 et R représente -OH et x et y sont tels que définis ci-dessus pour la formule 1 étant exclu, ledit procédé étant caractérisé en ce que l'on fait partiellement réagir un poly(acide anhydroaminodicarboxylique) de formule II: 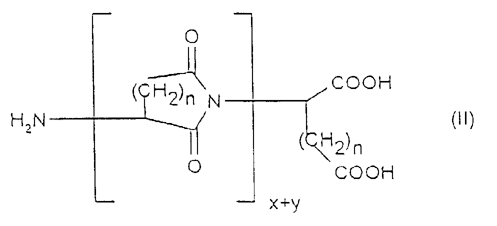 dans laquelle n, x et y sont tels que définis ci-dessus à la revendication 1, avec NH₃ ou un composé de formule III ou IV: 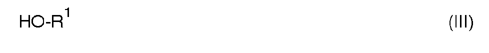 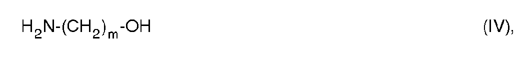 où
R¹ et m ont les significations données ci-dessus et on fait ensuite éventuellement réagir au cours d'une réaction de polymérisation le produit obtenu avec un ou plusieurs composés de formule V, VI ou VII: 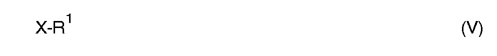 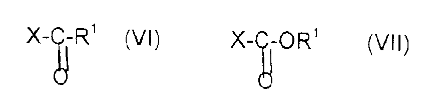 où R¹ est tel que défini ci-dessus et X est un groupe partant.

2. Procédé selon la revendication 1, caractérisé en ce que l'on prépare un copolymère acide aminodicarboxylique/ acide anhydroaminodicarboxylique de formule I dans laquelle
R représente NH-R2 et
m est 2 et
R¹ représente H, aryle, aralkyle, alkyle ou C₅-C₆-cycloalkyle, les groupes alkyle présentant de 1 à 22 atomes de carbone.

3. Procédé selon l'une des revendications 1 ou 2, caractérisé en ce que l'on prépare un copolymère acide aminodicarboxylique/acide anhydroaminodicarboxylique de formule I dans laquelle
R représente O-R¹ et
R^{l} représente aryle, aralkyle, alkyle ou C₅-C₆-cycloalkyle, les groupes alkyle présentant de 1 à 22 atomes de carbone.

4. Utilisation d'un polyamide selon la revendication 1, 2 ou 3, pour la préparation de formulations retard, biologiquement dégradables, à libération contrôlée du principe actif.

5. Utilisation d'un polyamide selon la revendication 1, 2 ou 3, pour l'encapsulage de substances biologiquement actives.

6. Utilisation d'un polyamide selon la revendication 1, 2 ou 3, en mélange avec d'autres polyamides biodégradables et/ou biocompatibles, ou des adjuvants physiologiquement acceptables pour la préparation de formes retard, biologiquement dégradables, à libération contrôlée du principe actif.
